# EUROPEAN PATENT APPLICATION

(11) **EP 1 832 661 A1**
(43) Date of publication of application: **12.09.2007**
(21) Application number: 06004849.3
(22) Date of filing: 09.03.2006
(51) Int. Cl.: C12Q 1/68

(54) **Method for the individual staging of tumor diseases**

(71) Applicant: Adnagen AG, 30853 Hannover-Langenhagen (DE)
(72) Inventor: Lustig, Michael, Dr., 27721 Ritterhude (DE); Böcher, Oliver, Dr., 8952 Schlieren- ZH (CH); Albert, Winfried, Dr., 31515 Wunstorf (DE)
(74) Representative: Pfenning, Meinig & Partner GbR

(57) **Abstract**

The present invention concerns a method for the individual staging of the tumor disease of an individual cancer patient, a method for the individual decision on the method of treatment as well as a method for treating a cancer patient as well as their use in the treatment of various cancer diseases like colorectal tumor, prostate tumor, breast tumor, lung tumor, as primary tumors, tumor relapse and/or metastases. The present invention further provides a new prognosis factor in cancer treatment. This inventive method includes the step of analyzing at least one disseminated tumor cell present in a sample taken from a patient for the expression of at least one mRNA of at least one of growth factors, growth factor receptors and tumor associated transcripts.

## Description

The present invention concerns a method for the individual staging of the tumor disease of an individual cancer patient, a method for the individual decision on the method of treatment as well as a method for treating a cancer patient as well as their use in the treatment of various cancer diseases like colorectal tumor, prostate tumor, breast tumor, lung tumor, as primary tumors, tumor relapse and/or metastases. The present invention further provides a new prognosis factor in cancer treatment.

In solid malignancies the probability of a relapse is related to the clinical staging of the primary tumor which is based on tumor characteristics like size, invasive growth, metastases in regional lymph nodes and presence of distant metastases at the time of primary surgery. However, the stratification of carcinoma patients into prognostic subgroups based on tumor characteristics is not sufficiently accurate to predict the individual patient status [Vlems, F.A., T.J. Ruers, C.J. Punt, T. Wobbes, and G.N. van Muijen. 2003. Relevance of disseminated tumour cells in blood and bone marrow of patients with solid epithelial tumours in perspective. Eur J Surg Oncol. 29:289-302].

Moreover, the early seed of metastatic cells in distant organs can contribute to metastatic relapse and is missed by traditional tumor staging [Müller, V., and K. Pantel. 2003. Clinical relevance of micrometastatic disease in patients with solid tumors. Am. J. Cancer. 2:77-86].

The prognostic value of blood-borne disseminated tumor cells (DTC) has been proven repeatedly and provides a new predictive clinical tool for improvement of therapy and survival in cancer:
(1) Meng, S., D. Tripathy, S. Shete, R. Ashfaq, B. Haley, S. Perkins, P. Beitsch, A. Khan, D. Euhus, C. Osborne, E. Frenkel, S. Hoover, M. Leitch, E. Clifford, E. Vitetta, L. Morrison, D. Herlyn, L.W. Terstappen, T. Fleming, T. Fehm, T. Tucker, N. Lane, J. Wang, and J. Uhr. 2004. HER-2 gene amplification can be acquired as breast cancer progresses. Proc Natl Acad Sci USA. 101:9393-8;
(2) Cristofanilli, M., G.T. Budd, M.J. Ellis, A. Stopeck, J. Matera, M.C. Miller, J.M. Reuben, G.V. Doyle, W.J. Allard, L.W. Terstappen, and D.F. Hayes. 2004. Circulating tumor cells, disease progression, and survival in metastatic breast cancer. N Engl J Med. 351:781-91.
(3) Cristofanilli, M., D.F. Hayes, G.T. Budd, M.J. Ellis, A. Stopeck, J.M. Reuben, G.V. Doyle, J. Matera, W.J. Allard, M.C. Miller, H.A. Fritsche, G.N. Hortobagyi, and L.W. Terstappen. 2005. Circulating tumor cells: a novel prognostic factor for newly diagnosed metastatic breast cancer. 1420-30 pp.

However,the expression profiling of blood-borne DTC revealed evidence of tumor cell heterogeneity within different patients and even in the course of the disease within the same patient.

The present invention aims to improve the staging of a cancer disease and thereby support the decision about therapy of a patient. It is thus the object of the present invention, to provide a method with which, in a reliable and repeatable manner, the characterization of a tumor with respect to clinical stage and possible therapy can be improved.

This object is solved by the method for the individual staging of a tumor according to claim 1 as well as by the method for individual decision on the method of treatment according to claim 28 and the method of treatment according to claim 29. Further improvements of the claimed methods are provided in the respective dependent claims.

According to the present invention, a method is provided, wherein at least one disseminated tumor cell present in a sample taken from a patient is analyzed for the expression of at least one mRNA of at least one of growth factors, growth factor receptors and tumor associated transcripts.

Preferably, the sample is analyzed for the expression of Epidermal Growth Factor Receptor (EGFR). Therein a decreasing level of expression of mRNA for EGFR indicates progression of the tumor disease, i.e. a high level of expression or presence of mRNA for EGFR indicates early stages of development of the tumor disease and a low level of expression or absence of mRNA for EGFR indicates late stages of development of the tumor disease. In particular, a high level of expression or presence of mRNA for EGFR indicates the absence of metastases (stage M0) and a low level of expression or absence of mRNA for EGFR indicates presence of metastases (stage M1)

The inventive method can be further improved by analyzing the DTC for the expression of mRNA for at least one of carcinoembryonic antigen (CEA), prostate specific antigen (PSA) and prostate specific membrane antigen (PSMA). Therein, an increasing level of expression of mRNA for at least one of CEA, PSA and PSMA concomitant with a decreasing level of expression of mRNA for EGFR indicates progression of the tumor disease.

CEA, PSA and PSMA as well as EpCAM, Her-2 and the tubulins are examples for tumor associated transcripts. The term "tumor associated transcripts" means all those transcripts that are related to the physiology or therapy of cancer or that are suitable to detect a tumor cell in the analyzed sample material.

The inventive method is in particularly useful for the staging and/or treatment of at least one of primary tumor, primary tumor relapse, local metastases and distant metastases in a cancer patient, in particular for the treatment of at least one of primary colorectal tumor, primary prostate tumor, primary breast tumor, primary lung tumor and metastases thereof.

Preferably the sample comprises at least one of the following: a body fluid, peripheral blood, sputum, ascites, lymph, urine, bone marrow, lymph nodes and biopsies. Further, the disseminated tumor cells may be isolated from the sample before analysis.

Further, the disseminated tumor cells (DTC) may be separated from the sample if necessary to improve the analysis, for example by positive or negative cell selection in liquid or solid phase. Suitable cell isolation methods comprise density gradient centrifugation, flow cytometry, micromanipulation, microdissection, e.g. with cells marked by fluorophores, or the method described in the international patent application PCT/EP02/05489 as published. The disclosure of this application with respect to the separation of disseminated tumor cells from a sample is fully included into the present application.

The present invention is particularly advantageous, because it is simple and easy as well as safe to take samples from a patient thus minimizing the risk for the patient. Such samples taken are a suitable material for analyzing DTC, as DTCs are usually present in such samples even after surgery of the primary tumor. It is possible to take a whole sample approach and analyzing the mRNA of the DTC in the whole sample or analyzing only the mRNA of DTC after separation of the DTC from other cells in the sample.

Thus, the present invention is a valuable tool for improvement of the individual characterization as well as the individual therapy of a patient as the prognostic value of viable disseminated tumor cells is superior in staging in comparison to the histopathologically determined expression status or the gene amplification status of the primary tumor or disseminated tumor cells.

Within this application, the term "disseminated tumor cell" (DTC) is equivalent to the terms "circulating tumor cells" or "micrometastases", which all have the same meaning of tumor cells disseminated from their tumor origin and possibly circulating, e.g. in the blood of patients.

Further, the term "ligand" within this application means all molecules that bind specifically to a specific target molecule. Ligands might be e.g. lectines, hormones, chemokines, cell activity modulating substances, cell adhesion molecules or the like.

### Cell selection

In the following, we describe the cell selection method according to PCT/EP02/05489, WO03/023057 A2, (equivalent to EP 1 409 727 A2).

In this method, firstly the DTC to be selected are marked by means of at least one or a combination of antibodies or antibody derivatives or ,by a bispecific antibody or antibody derivative. As a result, it is possible to mark, separate and hence concentrate, in particular the sought cells. This means that, in a first step, a preferably combined immunological detection or selection is effected. There is understood by antibody derivative in this application any type of altered antibody or antibody fragment which has a binding site, for example single-chain antibodies, antibody fragments, such as FAB fragments or recombinant antibodies. When "antibodies" are mentioned in the following, antibodies and/or antibody derivatives are always denoted.

In a second step, at least one marker, preferably a combination of markers, is then detected on a molecular-biological basis with a predefined combination of detection reagents, said markers being specific for the sought cells or being able to be found preferentially in the latter so that here again the sought cells are selected specifically. This therefore concerns here a preferably combined molecular-biological detection. The basic concept of this selection method is therefore to combine a detection via a combination of immunological parameters with a detection via a combination of molecular-biological parameters. Quite exceptional detection results are produced as a result, with which an advance is made into areas of detection which were not accessible previously. Therefore, concentrations of sought cells in blood samples down to one cell per 5 millilitres can even be detected.

A selection of the DTC precedes the detection of the markers via the binding of various antibodies to the sought cells, because the expression of special surface proteins differentiates cells of one type from cells of another type. Thus, for example the expression of special surface proteins differentiates tumor cells from non-transformed cells of this cell type.

Since the special pattern of the surface antigens for example in the case of tumor cells also differs from blood cell-typical patterns, tumor cells in the blood can be differentiated. In order to identify tumor cells, antibodies which specifically recognise these special surface proteins are used as tools. The specific antibody binding is exploited for various analysis and separation methods.

Due to the intensive binding of immunoglobulins, selected specially for this purpose, separation of the detected cells from non-detected cells is possible in addition to detection of cells via their surface epitope.

Instead of antibodies, any suitable specifier may be used to mark cells specifically.

Various separation principles are possible:

### 1. Separation principle based on liquid phase; e.g. flow cytometry:

For the flow-cytometric analysis, antibodies are coupled with fluorescence dyes. Cells pass a light source (laser) individually in a constant liquid flow. Upon illumination of the cells, the fluorescence dyes bound to the antibodies are excited and radiate light of specific wavelengths. The radiated light is detected and the measured signal is stored in a digital form. The light signal can be assigned to individual cells. The antibody-marked cell is detected thus and can now be separated from other cells. For separation, the cells are isolated into the smallest drops. After detection of the antibody-marked cell, the corresponding drop is directed into a collection receptacle. A concentration of this type can be effected for example by FACS flow cytometry. For example, concentrated cells with fluorescence-marked monoclonal antibodies against tumor-specific surface proteins are thereby incubated. The marked cells are washed twice with PBS and, subsequent thereto, 10⁷ cells are resuspended in 1 ml PBS. For the isolation of the tumor cells, a FACS vantage SE flow cytometer (Becton Dickinson) is used. Data recording, instrument control and data evaluation are effected via the CellQuest Software. The sorted cells are transferred into a 1.5 ml reaction vessel (filled with 1 ml BPS). The RNA can then be isolated as described later.

### 2. Separation principle based on solid phase; e.g. magnetic separation

Antibodies are coupled to para-magnetic particles for the magnetic separation. After introduction of the para-magnetic particles into a magnetic field, the particles migrate in the magnetic field. During the movement in this magnetic field, cells to which these coupled antibodies are bound, are entrained and separated from other cells.

For cell detection by means of magnetic particles, antibodies are thus coupled to para-magnetic particles which have a defined number of chemically activated sites on their surface. Coupling methods are known for example from James P. Gosling, Solid-phase Concepts and Design, in: R.F. Masseyeff, W. H. Albert N. A. Stoines (eds), Methods of Immunobogical Analysis, Vol. 1, VCH Verlagsgesellschaft mbH, Weinheim, pp. 507-529. The specificity of the separation is determined via the specificity of the antibodies. A blood sample containing target cells is mixed with antibody-coupled magnetic particles; then particles and blood are moved relative to each other, for example by "overhead rotation" of samples situated in a closed container or by movement of the particles due to changing magnetic fields. Those target cells which are detected by an antibody bound to the solid phase and hence securely bound, follow the movement of the particles. It is possible as a result, when applying a magnetic field, to withdraw the particles with the cells bound thereon from the blood (e.g. onto the wall of the separation vessel). The blood which is target cell-depleted in this manner can be exchanged for other solutions, the cells separated via magnetic particles remaining in situ until switching off/removal of the magnetic field and being available far further applications.

Specific antibody mixtures can be used advantageously for the detection of the tumor cells as will be shown later. By means of such antibody mixture disseminated tumor cells are detected preferentially, however with high specificity. This is based on the preferential expression of specific surface proteins which differentiates cancer cells from other cells.

The selection method according to PCT/EP02/05489 is substantially based furthermore on the fact that cell markers in the blood of patients are detected not for instance at an immunological or enzymatic level but by the fact that a molecular-biological marker, for example mRNA (messenger ribonucleic acid) of sought cells in a sample, for example in a blood sample, is detected.

Since individual markers are expressed differently in a therapy-dependent manner, a combination of tumor markers is advantageously tested in order to detect all the tumor cells circulating in the blood. As a result, tumor cells can also be detected when the expression of a specific marker is relatively low in a patient or in an illness stage, which otherwise could lead to a putatively negative result. The use of markers comes up against limits however usually for the reason that mononuclear blood cells have a background expression ("illegitimate transcription") which impedes exact analysis.

The expression of the genes mentioned in table 1 is detected as a marker, for example of tumors. The detection can thereby be implemented for one or two markers or also for any number of these tumor markers in combination with each other.

**Table 1:**

| Gene or gene product | Gene | Alternative designation |
|---|---|---|
| Human carcinoma-associated antigen GA733-2 gene | GA733-2 | GA733.2 |
| Human epidermal growth factor receptor (EGFR) gene | EGFR | EGFR |
| Human carcinoembryonic antigen (CEA) gene | CEA | CEA |
| Homo sapiens mucin 1 (MUC1) | MUC1 | CA15-3 |
| Homo sapiens C-erb B2/neu protein (ERBB2) gene | HER-2/neu | HER-2 |
| Homo sapiens claudin 7 (CLDN7), mRNA | claudin7 (CLDN7) | claudin7 |
| Alkaline phosphatase, placenta-like (Nago isozyme), (Germ-cell alkaline phosphatase), (PLAP-like) | ALPPL2 (GCAP) | PLAP |
| Homo sapiens gastrin-releasing peptide receptor (GPPR) gene | GRPR | GRPR |
| Homo sapiens high-mobility group (nonhistone chromosomal protein isoform I-C (HMGIC), mRNA | HMGIC | HMGI-C |
| Homo sapiens gene for cytokeratin 20 | CK20 | CK20 |
| Human MAGE-3 antigen (MAGE-3) gene | MAGE-3 | MAGE-3 |
| Homo sapiens stanniocalcin 1 (STC 1) gene | Stanniocalcin 1 (STC1) | stanniocalcin |

As an alternative to the above-presented separation principles according to PCT/EP02/05489, also any other separation principles from the state of the art, which are based on marking cells with ligands, antibodies or other specifiers, can be used.

In the following several examples of the method according to the present invention are provided.
- Fig. 1: shows the characteristics of colorectal cancer patients as analyzed in the first example (colorectal cancer);
- Fig. 2: shows the comparison of tumor associated gene expression in DTC in peripheral blood of the patients in fig. 1 (colorectal cancer);
- Fig. 3: shows the expression of tumor associated genes in DTC in peripheral blood of the patients in fig. 1 with and without metastatic disease (colorectal cancer);
- Fig. 4: shows the comparison of CEA expression in DTC with CEA serum protein levels (colorectal cancer) ;
- Fig. 5: shows the results of a further case study of the prognostic clinical value of DTC in colorectal cancer patient in follow-up samples;
- Fig. 6: shows the comparison of tumor associated gene expression in DTC in peripheral blood of prostate cancer patients and BPH patients.

In the following examples the following methods were used for separation and analysis of the DTC.

The basic procedure is common to all examples, said procedure comprising a step with immunological concentration of target cells.

### 1. Immunological concentration of the target cells from peripheral blood

Firstly, 5 ml peripheral blood sample were taken from the patients.

Furthermore, antibodies were coupled to magnetic particles. As antibodies, the antibodies MOC-31 (Novo-castra) and BerEP4 (DAKO) were used for the detection of colorectal cancer cells. For prostate cancer cells the antibodies 2D3 (Adnagen AG), a monoclonal antibody specific for EpCAM and 10E9 (Adnagen AG) specific for Her2 were used.

The magnetic particles were thereby used with a particle concentration of 4 x 10⁸ beads/ml (DYNABEADS, Pan Mouse IgG, Dynal Co.). The amounts of antibody used for the coating of the beads are shown in table 2.

**Table 2: Antibody mixture for cell separation**

| **Antigen** | **Clone** | **Concentration** | **Percentage in the respective mixture** |
|---|---|---|---|
| ***Prostate Cancer*** | | | |
| Her2 | 10E7 (Ad-naGen AG) | 25 µg/4*10⁸ beads | 50 % |
| EpCAM | 2D3 (Ad-naGen AG) | 25 *µ*g/4*10⁸ beads | 50 % |
| ***Colon Cancer*** | | | |
| Epithelial related antigen | MOC-31 (Novocastra) | 51,2 *µ*g/4*10⁸ beads | 50 % |
| EpCAM | BerEP-4 (DAKO) | 2,4 µg/4*10⁸ beads | 50 % |

4 x 10⁷ of the thus prepared magnetic particles were added to the blood sample.

After 30 min incubation in the overhead shaker, the magnetic particles, which occurred possibly as cell antibody magnetic particle complexes, were washed, by means of a magnetic particle concentrator (MPC^{®}-S, Dynal Co.), 3 times with PBS (phosphate buffer saline) and the adhering cells were subsequently treated corresponding to the subsequently described RNA isolation protocol.

### 2. mRNA isolation

mRNA from the separated DTC was isolated by means of oligo(dT)-coupled magnetic particles, Dynabeads® mRNA Direct^{TM} Micro Kit, (Dynal Co.). This isolation was performed corresponding to the manufacturer's instructions indicated in the kit. Isolation ended with an incubation for 5 min at 65° C (for prostate cancer) or 50° C (for colorectal cancer) .

### 3. Reverse transcription

A reverse transcription, in which the mRNA is transcribed into cDNA follows the isolation of the RNA.

The cDNA synthesis was effected at 37°C for one hour with subsequent inactivation of the reverse transcriptase by heating for 5 minutes at 95°C and subsequent cooling on ice. For this purpose, a Sensiscript Reverse Transcriptase Kit, Qiagen Co., Hilden was used according to the protocols indicated there (refer to table 3).

**Table 3: Reverse Transcription**

| **Components** | | | **Volume** Colorectal Cancer | **Volume** Prostate Cancer |
|---|---|---|---|---|
| **RT Mastermix** | *Sensiscript* Reverse Transcriptase Kit (Qiagen) | 10x Buffer RT | 4.0 µl | 2.0 µl |
| | | dNTPs | 4.0 µl | 2.0 µl |
| | | *Sensiscript* Reverse Transcriptase (SRT) | 2.0 µl | 1.0 µl |
| | RNase Inhibitor, 40 U/µl (Promega) | | 0.5 µl | 0.25 µl |
| **Sample** | mRNA/bead-complex or RNase-free H2O (as RT Control) | | 29.5 µl | 14.75 µl |
| **Total volume** | | | **40.0 µl** | **20.0 µl** |

The oligo(dT)-linker of the oligo(dT)-coupled particles serves simultaneously as primer for the reverse transcription.

### 4. PCR

Subsequent to the transcription of mRNA into cDNA, a polymerase chain reaction (PCR) is effected with β-actin as internal control.

The oligonucleotides cited in Table 4 were used as PCR primer for amplification of cDNA corresponding to different marker genes, as are indicated in the first column.

**Table 4: List of PCR primers**

| Primer name | Sequence 5' → 3' | PCR product |
|---|---|---|
| Tumour markers | | |
| GA733.2 sense | AATCGTCAATGCCAGTGTACTTCA | 395 bp |
| GA733.2 sense | TAACGCGTTGTGATCTCCTTCTGA | |
| EGFR sense | AGTCGGGCTCTGGAGGAAAAGAAA | 163 bp |
| EGFR antisense | GATCATAATTCCTCTGCACATAGG | |
| CEA sense | AGAAATGACGCAAGAGCCTATGTA | 231 bp |
| CEA antisense | AACTTGTGTGTGTTGCTGCGGTAT | |
| PSA sense | CAAAAGCGTG ATCTTGCTGG GTCGGC | 357 bp |
| PSA antisense | TGAACTTGCG CACACACGTC ATTGGAA | |
| PSMA sense | TGGTGCTGGC GGGTGGCTTC TTTCT | 449 bp |
| PSMA antisense | CACTAGATCG CCCTCTGGCA TTCCTTGA | |

| Internal control | | |
|---|---|---|
| actin sense | CTGGAGAAGAGCTACGAGCTGCCT | 114 bp |
| actin antisense | ACAGGACTCCATGCCCAGGAAGGA | |

| | | |
|---|---|---|
| Note: Using the above primers for EGFR, the presence of all EGFR variants are detected with the exception of those variants with deletions in exons 2 to 7. | | |

The length of the PCR product, which is produced by the primers indicated in column two, is indicated in column three. The PCR was implemented with the protocol indicated in Table 5. The PCR synthesis was effected in a 50 µl and 25 µl reaction batch.

**Table 5: Preparation of the multiplex PCR for the detection of colon cells**

| **Components** | | | **Volume** Colorectal Cancer | **Volume** Prostate Cancer |
|---|---|---|---|---|
| **PCR Master Mix** | *HotStarTaq Master Mix* Kit (Qiagen) | *HotStarTaq Master Mix* | 25.0 µl | 12.5 µl |
| | | Distilled water | 13.0 µl | 4.5 µl |
| | *PrimerMix Colon*/*Prostate* | | 4.0 µl | 4.0 µl |
| **Samples** | cDNA or RT Control or Negative Control (water/C-) or *Positive Control* (C+) each: | | 8.0 µl | 4.0 µl |
| **Total volume** | | | 50.0 µl | 25.0 µl |

Table 6 indicates a list of the specific primer combination and primer concentrations as end concentration in the PCR batch. In the following examples for the various tumour types, colorectal cancer and prostate cancer respectively, a multiplex combination for these primers is shown by way of example.

**Table 6: List of the specific primer combinations and primer concentration (end concentration in the PCR batch)**

| Marker Primer | Colorectal Cancer | Prostate cancer |
|---|---|---|
| GA733.2 sense | 100 nM | 750 nM |
| GA733.2 antisense | 100 nM | 750 nM |
| EGFR sense | 750 nM | |
| EGFR antisense | 750 nM | |
| CEA sense | 750 nM | |
| CEA antisense | 750 nM | |
| PSA sense | | 200 nM |
| PSA antisense | | 200 nM |
| PSMA sense | | 500 nM |
| PSMA antisense | | 500 nM |
| β-actin sense | 100 nM | 100 nM |
| β-actin antisense | 100 nM | 100 nM |

The PCR conditions (cycle number, cycle process etc.) are given in Tables 7 and 8.

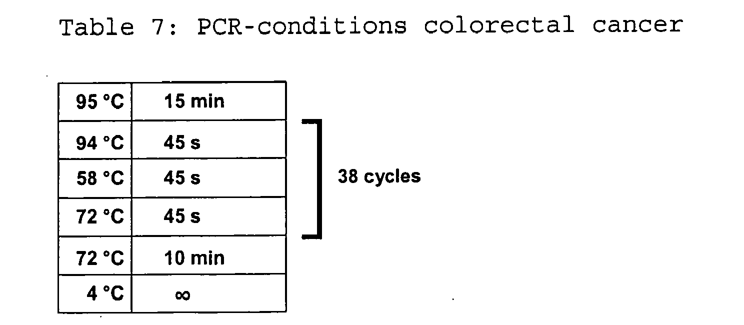

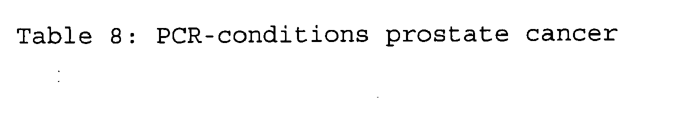

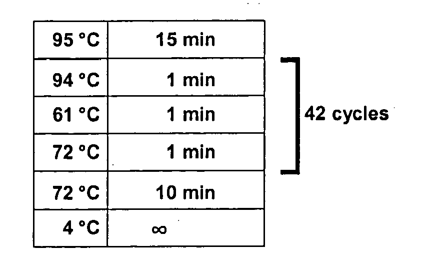

The thus produced amplificates of the cDNA were separated electrophoretically by means of a bioanalyser 2100 (Agilent Co.). For this purpose, 1 µl of the PCR product was separated in the bioanalyser on a DNA chip (1000) and the separation result was documented electronically.

### Example 1

### Expression of tumor-associated genes in blood samples of colorectal cancer patients

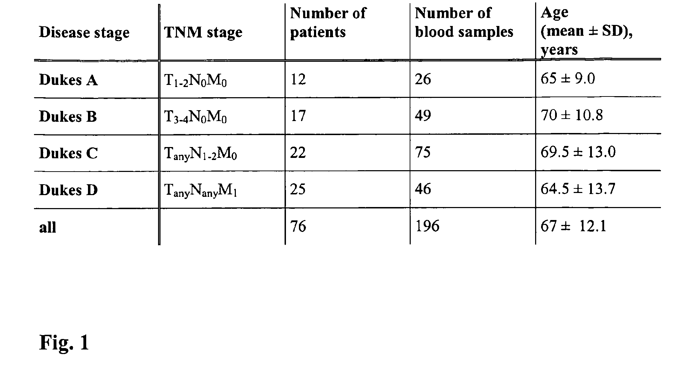

In a first example, 196 peripheral blood samples collected from 76 colorectal cancer patients with different tumor stages were analyzed (Table in Fig. 1). 12 patients were staged Dukes A, 17 patient were staged Dukes B, 22 patients were staged Dukes C and 25 patients suffered from metastatic disease (Dukes D). The overall mean age was 67 years (± 12.1). Mean follow-up time was 47 weeks (range 6-143 weeks). After blood withdrawal the samples were incubated at 4°C and processed within four hours for DTC (disseminated tumor cells) separation and expression analysis.

Expression analysis in DTC separated from the samples of peripheral blood of the carcinoma patients was performed by the DTC detection assay using the tumor-associated transcripts CEA, EGFR and GA733-2.

The expression of tumor-associated genes CEA, EGFR, and GA733-2 was assessed in blood samples of patients with tumor stage Dukes A, B, and C collected prior to surgery and post surgery. Results were compared with tumor-associated gene expression in blood samples collected from patients suffering from metastatic disease (Dukes D) to evaluate disease progression and tumor stage dependence.

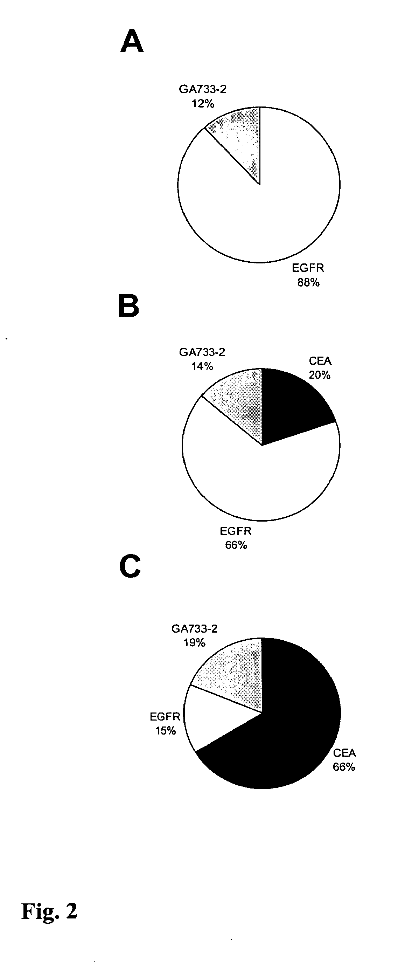

The result of this analysis is shown in Fig. 2. Blood samples (n=196) were collected (A) prior to surgery, (B) post surgery, and (C) from metastatic patients (M1) . Samples were analyzed by the DTC detection assay and expression of the tumor-associated genes CEA, EGFR, and GA733-2 were assessed. Differences between groups (prior to surgery, post surgery, M1, and healthy donors; n=106) were statistically compared for each tumor-associated gene (EGFR p=0.001 prior to surgery vs. M1 and post surgery vs. M1, p<0.0001 healthy donors vs. M1; CEA p=0.002 prior to surgery vs. M1 and post surgery vs. M1, p<0.0001 healthy donors vs. M1; GA733-2 p=1.00 prior to surgery vs. M1 and post surgery vs. M1, p=0.016 healthy donors vs. M1).

The comparison analysis revealed an EGFR expression in 88 % (p=0.001), in 66 % (p=0.001), and in 15 % (p<0.0001) of blood samples collected prior to surgery, post surgery and from metastatic patients, respectively. On the other hand, no expression of CEA was detected in blood samples collected prior to surgery (p=0.002), while CEA expression was detected in 20 % (p=0.002), and in 66 % (p<0.0001) of blood samples collected from patients post surgery and from metastatic patients, respectively. GA733-2 was expressed in 12 % (p=1.00), in 14 % (p=1.00), and in 19 % (p=0.016) of blood samples collected prior to surgery, post surgery, and from metastatic patients, respectively.

Hence, a highly statistically significant decline in EGFR expression and a concomitant increase in CEA expression was observed during progression reflecting changes of gene expression profiles in DTC. No such correlation was observed with respect to GA733-2, whose expression was more evenly distributed.

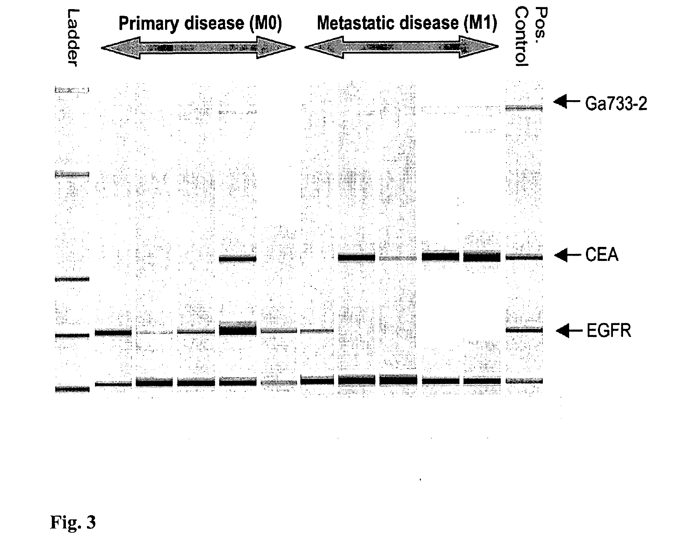

Examples of the before mentioned tumor-associated gene expression distribution are shown in Figure 3. FIGURE 3 shows the expression of tumor-associated gens in DTC in peripheral blood of colorectal cancer patients with and without metastatic disease. Blood samples of colorectal cancer patients with and without metastatic disease were analyzed by the above mentioned DTC detection assay. Amplified DNA fragments of the tumor markers GA733-2 (383 bp), CEA (226 bp) and EGFR (161 bp) are shown. DNA fragments were analyzed by high voltage microfluidic gel electrophoresis with a Bioanalyzer 2100 (Agilent Technologies).

### Example 2

### Clinical value of CEA expression in DTC in comparison with CEA serum protein levels

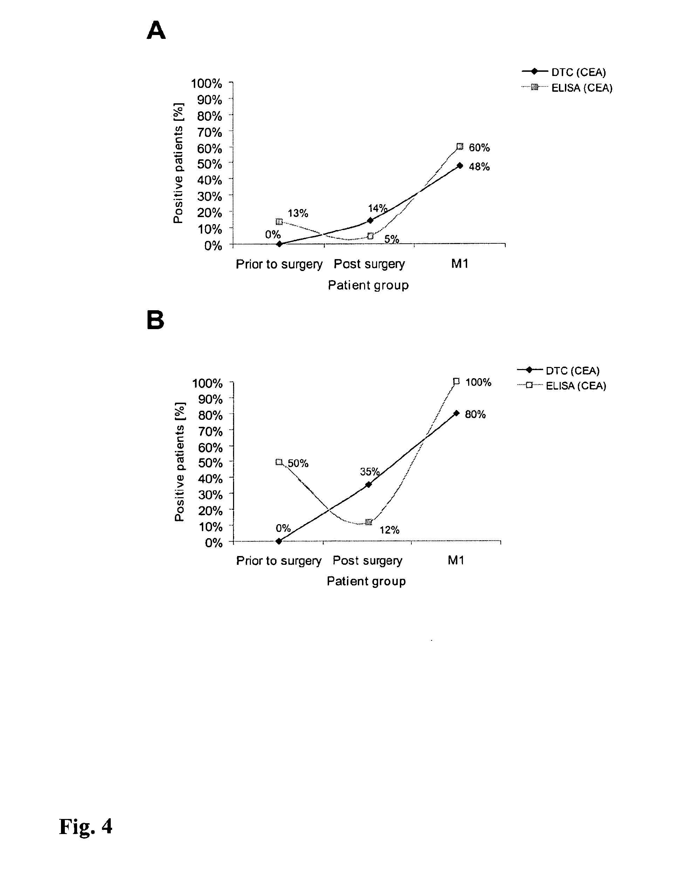

The particular role of CEA with respect to its clinical value was further evaluated by comparing CEA expression in DTC with CEA serum protein levels as shown in fig. 4. Blood samples of (A) all patients (n=196) and (B) DTC positive colorectal cancer patients (n=40) were collected prior to surgery, post surgery, and from metastatic patients (M1) and analyzed by the DTC detection assay. Expression of the tumor-associated gene CEA was assessed in DTC and compared with CEA serum protein levels. A CEA concentration above 5 ng/mL serum was considered to be elevated. Differences between groups (prior to surgery, post surgery, M1, and healthy donors; n=106) were statistically compared for the CEA gene (p<0.0001 prior to surgery vs. M1 and healthy donors vs. M1, p=0.005/0.011 post surgery vs. M1).

CEA expression in DTC was not detectable in blood samples collected prior to surgery (p<0.0001) whereas CEA serum protein levels were detected in up to 13 % of patients (n=30) which is attributable to CEA protein production of the primary tumor (Figure 4A). In blood samples (n=43) collected post surgery elevated CEA serum protein levels were detected in 5 % whereas CEA expression in DTC was detected in up to 14 % (p=0.005) of samples. In blood samples collected from metastatic patients elevated CEA serum protein levels were detected in 60 % and CEA expression in DTC in 48 % (p<0.0001) of patients (n=25) showing congruent high expression in DTC and high elevated CEA serum protein levels as well. In DTC positive patients the observed correlation was even more clearly demonstrated (Figure 4B). Elevated CEA serum protein levels were measured in 50 %, in 12 %, and in 100 % of samples collected prior to surgery (n=8), post surgery (n=17), and from metastatic patients (n=15), respectively. In contrast, CEA expression was detected in 0 % (p<0.0001), in 35 % (p=0.011), and in 80 % (p<0.0001) of samples collected prior to surgery, post surgery, and from metastatic patients, respectively.

The results show a linear increase of CEA expression during disease progression whereas no such correlation was observed with respect to the elevation of CEA serum protein levels. Detection of CEA expression in DTC seems to precede the occurrence of CEA serum protein elevation and might be predictive as an early indicator of relapse.

### Example 3

### Case study: Prognostic clinical value of DTC.

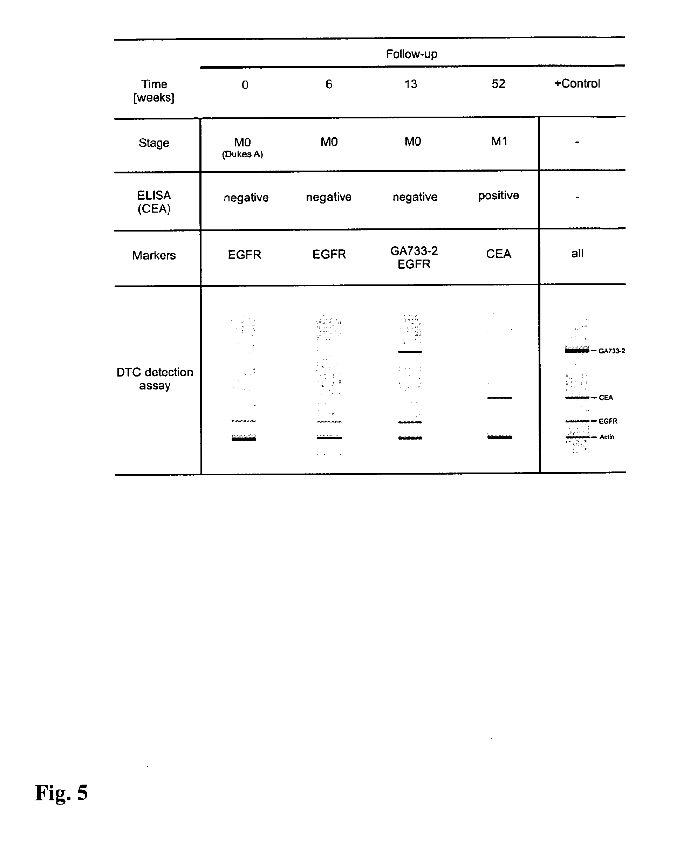

Fig. 5 shows a follow-up study of a colorectal cancer patient with tumor stage Dukes A. Blood samples were collected prior to surgery and in regular time intervals up to 52 weeks post surgery. Detection of DTC by expression profiling was compared with elevation of CEA serum protein levels. cDNA derived from colorectal cancer cell lines served as positive control (+Control). A CEA concentration above 5 ng/mL serum was considered to be elevated.

In this colorectal cancer patient with tumor stage Dukes A DTC were detected from the beginning of monitoring while the CEA serum protein levels were always below 5 ng/mL giving no indication of potential tumor relapse (Figure 5). After 52 weeks the patient was diagnosed with multiple liver metastases. With respect to the tumor-associated genes EGFR and CEA an expression shift was observed. While EGFR expression was detected at the beginning of monitoring, no expression was observed in blood samples collected after 52 weeks. On the other hand, expression of CEA was not detectable within the first 13 weeks of monitoring but only in blood samples collected after 52 weeks.

The results indicate that detection of DTC by expression profiling using tumor-associated genes might serve as a valuable factor to predict potential formation of clinical metastases earlier than the elevation of CEA in serum.

### Example 4

### Expression of tumor-associated genes in blood samples of prostate cancer patients

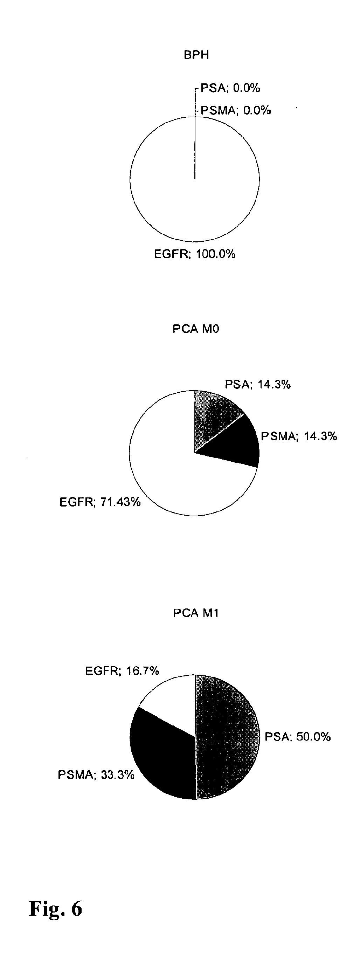

FIGURE 6 shows a comparison analysis of tumor-associated gene expression in DTC in peripheral blood of prostate cancer patients.

Blood samples (n=48) were collected from patients with benign prostate hyperplasia (BPH), prostate cancer patients with stage M0, and from metastatic patients (M1). Samples were analyzed by the above mentioned DTC detection assay and expression of the tumor-associated genes EGFR, PSA and PSMA in the isolated DTC were assessed.

Peripheral blood samples from 28 prostate cancer patients with different tumor stages and 20 patients with a benign prostate hyperplasia were analyzed. 17 prostate cancer patients were staged M0 without metastases, and 11 patients suffered from metastatic disease (M1). After blood withdrawal the samples were incubated at 4°C and processed within four hours.

The expression of tumor-associated genes EGFR, PSA and PSMA was assessed in blood samples of patients with tumor stage M0 and were compared with tumor-associated gene expression in DTC from blood samples collected from patients suffering from metastatic disease (M1) to evaluate a disease progression and tumor stage dependence.

The comparison analysis revealed an EGFR expression in 100 %, in 71.4 %, and in 16.7 % of blood samples of BPH, M0 and M1 patients with detectable disseminated prostate cells in the blood, respectively (Figure 5). On the other hand, no expression of PSA and PSMA was detected in blood samples collected from BPH patients, while PSA expression was detected in 14.3 %, and in 50 % of blood samples containing disseminated prostate cells collected from prostate cancer patients M0 and M1, respectively. PSMA expression was detected in 14.3 %, and in 33.3 % of blood samples containing disseminated prostate cells collected from prostate cancer patients M0 and M1, respectively.

Hence, an obvious decline in EGFR expression and a concomitant increase in PSA and PSMA expression was observed during progression reflecting changes of gene expression profiles in DTC.

One EGFR positive patient that was initially classified as BPH patient was re-classified in a following clinical examination as prostate cancer patient. Even if this is an obversation made in an individual case it supports the diagnostic potential of EGFR as it is shown in Figure 6.

### Conclusion

Whereas state of the art staging parameters are based entirely on the characteristics of the primary tumor and metastases, a staging parameter based on the detection of DTC is not in clinical use. Hence, the determination of molecular DTC characteristics, as they are subject of the present invention, are not in use either.

For colorectal cancer, we examined the disease progression and tumor stage dependent expression of the tumor-associated genes EGFR, CEA, and GA733-2 in DTC. In a comparison analysis the expression of EGFR was found to be high (88 %) in DTC detected prior to surgery compared to the detection levels post surgery (66 %) and in metastatic patients (15 %).

Our results for colorectal cancer are in-line with our findings in prostate cancer. In a simultaneous RT-PCR analysis of EGFR, PSA and PSMA we found only 16.7 % EGFR amplificates in metastatic patients (M1) compared to 71.4 % in M0 patients and 100% in patients with BPH what can be a pre-stage of prostate cancer.

The above presented data of prostate and colorectal cancer show clearly that EGFR expression in DTC is suitable as a new staging parameter. The presence of EGFR mRNA in DTCs indicates stage M0 and even benign pre-stages of cancer, whereas the absence of EGFR mRNA in DTCs indicates stage M1.

Up to now the EGFR expression in blood was described as an indicator for the presence of DTCs. In particular, Gazzaniga, P., I. Nofroni, O. Gandini, I. Silvestri, L. Frati, A.M. Agliano, and A. Gradilone. 2005. Tenascin C and epidermal growth factor receptor as markers of circulating tumoral cells in bladder and colon cancer. Oncol Rep. 14:1199-202 (2005) showed a positive correlation of EGFR expression in the blood and increased relapse rates for colorectal and bladder cancer. Consequently the conclusion of this report is contradictory to ours, namely that expression of EGFR mRNA in blood is a marker for stage M1.

Further, the detection of PSA, PSMA as well as CEA mRNA in DTC positively correlates with the disease stage - as it was deducible so far from analyses of blood and of cancer tissue. But surprisingly EGFR expression in DTC shows opposite characteristics.

Only if DTCs are analyzed EGFR expression bears the diagnostic potential that is subject of our invented method. Therefore the use of DTCs for the determination of the cancer stage is per se an inventive step whenever expression of a specific mRNA by DTCs is not predictable from expression analyses that used samples other than DTCs.

In contrast to EGFR all other analyzed markers were positive indicators for stage M1. Only EGFR can be used as positive marker for stage M0. The prediction accuracy can be improved by combining the information of different expression markers.

## Claims

1. Method for the individual staging of the tumor disease of an individual cancer patient, including the step of:
analyzing at least one disseminated tumor cell present in a sample taken from a patient for the expression of at least one mRNA of at least one of growth factors, growth factor receptors and tumor associated transcripts.

2. Method according to claim 1, wherein the step of analyzing includes the step of analyzing for the expression of Epidermal Growth Factor Receptor (EGFR).

3. Method according to claim 2, wherein a decreasing level of expression of mRNA for EGFR indicates progression of the tumor disease.

4. Method according to one of claims 2 and 3, wherein a high level of expression or presence of mRNA for EGFR indicates early stages of development of the tumor disease and a low level of expression or absence of mRNA for EGFR indicates late stages of development of the tumor disease.

5. Method according to one of claims 2 to 4, wherein a high level of expression or presence of mRNA for EGFR indicates the absence of metastases and a low level of expression or absence of mRNA for EGFR indicates presence of metastases.

6. Method according to one of claims 2 to 5, wherein the step of analyzing further includes the step of analyzing for the expression of mRNA for at least one of carcinoembryonic antigen (CEA), prostate specific antigen (PSA) and prostate specific membrane antigen (PSMA).

7. Method according to claim 6, wherein an increasing level of expression of mRNA for at least one of CEA, PSA and PSMA concomitant with a decreasing level of expression of mRNA for EGFR indicates progression of the tumor disease.

8. The method according to one of the preceding claims, wherein the tumor disease is one of colorectal cancer and prostate cancer.

9. The method according to one of the preceding claims, wherein the sample comprises at least one of the following: a body fluid, peripheral blood, sputum, ascites, lymph, urine, bone marrow, lymph nodes and biopsies.

10. The method according to one of the preceding claims, wherein disseminated tumor cells are isolated from the sample for use in the analyzing step.

11. The method according to claim 10, wherein the tumor cells are isolated by positive cell selection.

12. The method according to claim 10, wherein the tumor cells are isolated by one of negative cell selection and depletion of cells different than disseminated tumor cells.

13. The method according to one of claims 10 to 12, wherein the isolation of cells is effected in liquid phase or on a solid phase.

14. The method according to one of claims 10 to 13, wherein the disseminated tumor cells are isolated from the sample by density gradient centrifugation.

15. The method according to claim 11, wherein isolating the disseminated tumor cells from the sample includes the following steps:
mixing the sample with at least one specifier of tumor cell-associated markers and separating the cells marked with at least one of said at least one specifier.

16. The method according to claim 12, wherein isolating the disseminated tumor cells from the sample includes the following steps:
mixing the sample with at least one specifier of non-tumor cell-associated markers and separating the cells marked with at least one of said at least one specifier.

17. The method according to one of claims 15 and 16, wherein the sample is mixed with at least two specifiers.

18. The method according to one of claims 15 to 17, wherein at least one of the at least one specifier is coupled to solid phases in order to separate the cells from the sample.

19. The method according to one of claims 15 to 17, wherein at least one of the at least one specifier is marked with fluorophores, chromophores and/or other microscopically specifiable particles and the separation of the marked cells from the sample is effected by means of flow cytometry, micromanipulation or microdissection.

20. The method according to one of claims 15 to 17, wherein at least one of the at least one specifier is coupled to magnetic or paramagnetic particles and
in order to separate the marked cells from the sample, the magnetic or paramagnetic specifier-coupled particles are separated magnetically from the sample after mixing with the sample.

21. The method according to claim 15, wherein at least one of the at least one specifier has a binding site which binds to tumor cells of one or more tumor types or sub-types.

22. The method according to one of claims 15 to 21, wherein an antibody and/or ligand is used as specifier.

23. The method according to claim 15, wherein isolating the disseminated tumor cells from the sample includes the following steps:
mixing the sample with at least one antibody, that binds with its binding site to an epitope of disseminated tumor cells and isolating the cells marked with the at least one antibody from the sample.

24. The method according to claim 15, wherein isolating the disseminated tumor cells from the sample includes the following steps:
mixing the samples with (a) a predetermined combination of at least two antibodies and/or antibody derivatives, that bind with their binding sites to different epitopes of the cells to be isolated and/or with (b) at least one bispecific antibody an/or antibody derivative, that binds with its two binding sites to different epitopes of the cells to be isolated, wherein the binding sites bind to tumor cells, and
isolating the cells marked with at least one of the antibodies and/or antibody derivatives from the sample.

25. The method according to one of the preceding claims, wherein before or together with analyzing the mRNA of the disseminated tumor cells for the expression of at least one mRNA of at least one of growth factors, growth factor receptors and tumor associated transcripts, the separated tumor cells are analyzed with at least one molecular-biological reagent for the expression of at least one mRNA sequence, the expression of which is effected at least in disseminated tumor cells.

26. The method according to the preceding claim, wherein before or together with analyzing the mRNA of the disseminated tumor cells for the expression of at least one mRNA of at least one of growth factors, growth factor receptors and tumor associated transcripts, the separated tumor cells are analyzed with a predetermined combination of at least two molecular-biological detection reagents for at least two mRNA sequences for the expression of at least one mRNA sequence of said predetermined combination of at least two mRNA sequences, the expression of which is effected at least in disseminated tumor cells.

27. The method according to one of the preceding claims, wherein at least segments of mRNA to be analyzed are amplified and/or detected using polymerase chain reaction (PCR), ligase chain reaction (LCR), nucleic acid sequence based amplification (NASBA), reverse transcription polymerase chain reaction (RT-PCR), linear amplification, transcription mediated amplification (TMA); loop-mediated isothermal amplification (LAMP) and/or hybridization methods.

28. A method for individual decision on the method of treatment of an individual cancer patient, wherein at least one mRNA of at least one of growth factors, growth factor receptors and tumor associated transcripts is individually analyzed according to one of claims 1 to 27 and wherein it is individually decided on the method of treatment on the basis of the detected expression level of the analyzed mRNA.

29. Method of treating an individual cancer patient, wherein at least one mRNA of at least one of growth factors, growth factor receptors and tumor associated transcripts is individually analyzed according to one of claims 1 to 27 and wherein the patient is individually treated on the basis of the detected expression level of the analyzed mRNA.

30. Use of a method according to one of claims 1 to 29 for the treatment of at least one of primary tumor, primary tumor relapse, local metastases and distant metastases in a cancer patient.

31. Use of a method according to one of claims 1 to 30 for the treatment of at least one of primary colorectal tumor, primary prostate tumor, primary breast tumor, primary lung tumor and metastases thereof.


SEQUENCE LISTING

<110> Adnagen AG

<120> Method for the individual staging of tumor diseases

<130> 060334

<140> 060334
<141> 2006-03-09

<160> 12

<170> PatentIn Ver. 2.1

<210> 1
<211> 24
<212> DNA
<213> Artificial Sequence

<220>
<223> Description of Artificial Sequence:
      Oligonucleotide

<400> 1
agtcgggctc tggaggaaaa gaaa                                        24


<210> 2
<211> 24
<212> DNA
<213> Artificial Sequence

<220>
<223> Description of Artificial Sequence:
      Oligonucleotide

<400> 2
gatcataatt cctctgcaca tagg                                        24


<210> 3
<211> 26
<212> DNA
<213> Artificial Sequence

<220>
<223> Description of Artificial Sequence:
      Oligonucleotide

<400> 3
caaaagcgtg atcttgctgg gtcggc                                      26


<210> 4
<211> 27
<212> DNA
<213> Artificial Sequence

<220>
<223> Description of Artificial Sequence:
      Oligonucleotide

<400> 4
tgaacttgcg cacacacgtc attggaa                                          27

<210> 5
<211> 25
<212> DNA
<213> Artificial Sequence

<220>
<223> Description of Artificial Sequence:
      Oligonucleotide

<400> 5
tggtgctggc gggtggcttc tttct                                            25

<210> 6
<211> 28
<212> DNA
<213> Artificial Sequence

<220>
<223> Description of Artificial Sequence:
      Oligonucleotide

<400> 6
cactagatcg ccctctggca ttccttga                                         28

<210> 7
<211> 24
<212> DNA
<213> Artificial Sequence

<220>
<223> Description of Artificial Sequence:
      Oligonucleotide

<400> 7
aatcgtcaat gccagtgtac ttca                                             24

<210> 8
<211> 24
<212> DNA
<213> Artificial Sequence

<220>
<223> Description of Artificial Sequence:
      Oligonucleotide

<400> 8
taacgcgttg tgatctcctt ctga                                             24

<210> 9
<211> 24
<212> DNA
<213> Artificial Sequence

<220>
<223> Description of Artificial Sequence:

Oligonucleotide

<400> 9
agaaatgacg caagagccta tgta                                        24


<210> 10
<211> 24
<212> DNA
<213> Artificial Sequence

<220>
<223> Description of Artificial Sequence:
      Oligonucleotide

<400> 10
aacttgtgtg tgttgctgcg gtat                                        24


<210> 11
<211> 24
<212> DNA
<213> Artificial Sequence

<220>
<223> Description of Artificial Sequence:
      Oligonucleotide

<400> 11
ctggagaaga gctacgagct gcct                                        24


<210> 12
<211> 24
<212> DNA
<213> Artificial Sequence

<220>
<223> Description of Artificial Sequence:
      Oligonucleotide

<400> 12
acaggactcc atgcccagga agga                                        24